Europäisches Patentamt

European Patent Office

Office européen des brevets

(19)

(11) Publication number: **0 098 716**
**B1**

(12) **EUROPEAN PATENT SPECIFICATION**

(45) Date of publication of patent specification: **25.03.87**

(21) Application number: **83303627.0**

(22) Date of filing: **23.06.83**

(51) Int. Cl.⁴: **G 01 N 33/22**

(54) **Calorimeters for and methods of monitoring calorific values of gases.**

(30) Priority: **02.07.82 US 394955**

(43) Date of publication of application:
**18.01.84 Bulletin 84/03**

(45) Publication of the grant of the patent:
**25.03.87 Bulletin 87/13**

(84) Designated Contracting States:
**DE FR GB IT**

(56) References cited:
**EP-A-0 031 145**
**DD-A-2 004 875**
**US-A-3 597 345**
**US-A-4 125 018**
**US-A-4 329 873**

(73) Proprietor: **THE BABCOCK & WILCOX COMPANY**
**1010 Common Street P.O. Box 60035**
**New Orleans Louisiana 70160 (US)**

(72) Inventor: **Bohl, Thomas L.**
**306 Hyder Drive**
**Madison Ohio 44057 (US)**
Inventor: **Pocock, Robert E.**
**1058 W. Mill Drive**
**Highland Heights Ohio 44148 (US)**
Inventor: **Zimmerlin, Sharon L.**
**180 Elm Court**
**Chagrin Falls Ohio 44022 (US)**

(74) Representative: **Cotter, Ivan John et al**
**D. YOUNG & CO. 10 Staple Inn**
**London WC1V 7RD (GB)**

EP 0 098 716 B1

Courier Press, Leamington Spa, England.

## Description

This invention relates to calorimeters for and methods of continuously monitoring the calorific value of gases.

Various techniques of calorimetry are known. One such technique, known as water-flow calorimetry, relies on the principle of operation that heat is transferred by the combustion of a continuous flowing gas to continuously flowing water. The amount of water and the volume of gas combusted are known and the rise of temperature in the water is measured. A disadvantage of this technique is in response time, losses due to heat exchange with surroundings and the difficulty of ensuring complete transfer of heat from the combustion products to the water.

Another technique known as differential expansion calormetry has been utilised by the Sigma Instrument Company Limited and is disclosed in a publication by that company entitled "Mark II Recording Calorimeter for Trouble-Free Recording". In this technique the heat capacity of specific gases is obtained by venting the hot combustion products through two concentrically mounted metal tubes. The differential expansion of the tubes has a direct relationship to the thermal input of the gas being burned. A disadvantage of this technique also is heat exchange with the surroundings, particularly where the instrument might be heated unequally.

European Patent Application Publication No. EP—A—0 031 145 discloses a method and apparatus for determining the heating (calorific) value of gaseous fuels in which a mixture of fuel and air of known proportions is combusted and the products of combustion are analysed by an electrochemical sensor for the presence of residual oxygen. The relative proportions of fuel and air are then adjusted until the oxygen analysis of the combustion products indicates that the mixture is stoichiometric. The heating value of the fuel is then determined by a microprocessor using a relationship between the heating values of the constituents of the fuel and the amount of oxygen required for stoichiometric combustion.

According to the present invention there is provided a calorimeter for continuously monitoring the calorific value of a test gas, the calorimeter comprising:

first flow means for supplying a known volume of test gas;

second flow means for supplying a known volume of oxygen-containing gas having a known oxygen amount;

a burning chamber connected to the first and second flow means for receiving the test and oxygen-containing gases and completely burning the test gas with an amount of oxygen of the oxygen-containing gas to produce a combustion product;

heating means for heating the burning chamber to produce burning;

an electrochemical oxygen measuring cell connected to the chamber for receiving the combustion product and measuring any remaining oxygen in the product; and

circuit means connected to the cell;

characterised in that:

the first and second flow means are constant flow means for respectively supplying constant known volumes of test gas and oxygen-containing gas;

the burning chamber is a catalytic burning chamber;

the constant known volumes of the test gas and the oxygen-containing gas are set so that the combustion product has residual oxygen therein; and

the circuit means is operative to generate a value proportional to the difference between the known volume of oxygen and the amount of remaining oxygen, which value is proportional to the amount of oxygen consumed in the combustion chamber, the amount of oxygen consumed being proportional to the calorific value of the test gas.

The invention also provides a method of continuously monitoring the calorific value of a test gas, the method comprising:

supplying the test gas at a known flow rate to a combustion chamber;

supplying an oxygen-containing gas having a known oxygen amount to the combustion chamber at a known flow rate;

heating the combustion chamber to burn the test gas to consume an amount of oxygen and produce a combustion product;

measuring the amount of any remaining oxygen in the combustion product; and

determining the calorific value of the test gas;

characterised in that:

the test gas and the oxygen-containing gas are supplied at constant known flow rates;

the combustion chamber is a catalytic combustion chamber;

the constant known flow rates of the test gas and the oxygen-containing gas are set so that the combustion product has residual oxygen therein; and

the calorific value of the test gas is determined from the measured amount of oxygen consumed in the catalytic burning chamber, which amount of oxygen consumed is derived from the difference between the known volumetric flow of oxygen into the chamber and the amount of residual oxygen in the combustion product.

A preferred embodiment of the present invention described hereinbelow takes advantage of the

2

phenomenon that the amount of oxygen required for combustion is proportional to the calorific value of a gas. See Gas Calorimetry, C. G. Hyde and M. W. Jones, Ernest Benn Limited, London, 1960, page 411. The preferred embodiment takes advantage of this principle and utilises an electrochemical measuring cell, specifically a $ZrO_2$ fuel cell, to measure an amount of oxygen remaining in a known sample of oxygen-containing gas after the oxygen-containing gas has been used to oxidise a test gas. Details of this fuel cell are not given here but can be discovered for example from US Patent 3 597 345 (Hickam et al).

According to the preferred embodiment, the calorific value of a test gas can be obtained in a real-time measurement which can be utilised for a control function or simply as an indicator value.

For complete oxidation of the test or sample gas, a catalytic burning chamber is utilised which is elevated to a high temperature of about 815°C (1500°F). This catalytic burning is utilised, rather than flame burning, since flame burning can seldom achieve complete oxidation due to the masking of unreacted molecules by reaction products.

The preferred calorimeter described below is simple in design, rugged in construction and economical to manufacture.

The invention will now be further described, by way of illustrative and non-limiting example, with reference to the accompanying drawings, in which:

Figure 1 is a graph showing the relationship between calorific value in MJ (BTU) versus cubic metres (cubic feet) of oxygen required for combustion; and

Figure 2 is a schematic representation of a calorimeter embodying the invention.

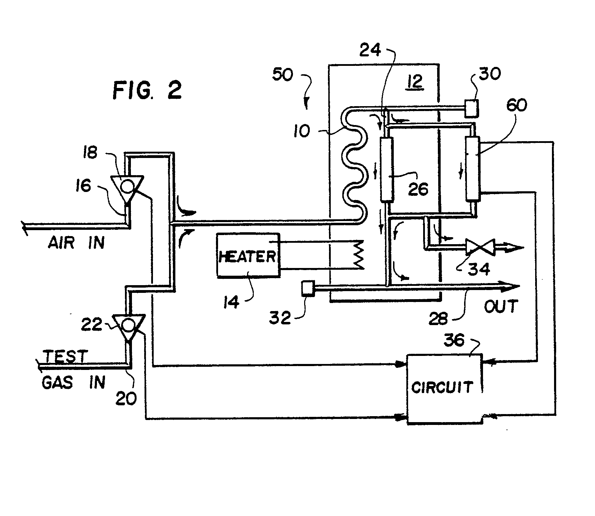

Referring to the drawings, Figure 2 shows a calorimeter 50 which utilises a measuring cell 60 to measure the amount of remaining oxygen in a mixture of combustion products in a catalytic burning chamber 10 defined in a heated block 12. The heated block 12 is heated by a heating element schematically represented at 14.

Oxygen-containing gas, preferably air, is conducted via an inlet line 16 to a constant flow pump 18. The constant flow pump 18 may be any type which can accurately be regulated for supplying a constant volumetric flow rate, such as a reciprocating cylinder pump having appropriate switching valves. The operation of the constant flow pump 18 can also be regulated to ensure constant pressure so that no compensation is necessary for changes in specific gravity of the air. A sample or test gas, which may for example be a fuel or other oxidisable gas, is conducted via a test gas inlet line 20 to another constant flow pump 22. The volumetric flow rate of the sample gas, as well as that of the air, is thus known. The two gases are mixed and directed to the catalytic burning chamber 10 which is serpentine for a complete combustion of the combustible elements in the test sample. The heating means 14 is operable to maintain the heated block 12 at a temperature of about 815°C (1500°F). From the catalytic combustion or burning chamber 10 the products, with now reduced oxygen, are supplied over a line 24. The flow then is divided between a bypass line going to the measuring cell 60 and a line 26. After the cell 60 the gas is reunited and directed via a line 28 to an outlet. Inspection plugs 30, 32 may be provided for inspecting various lines in the system and a test gas port 34 may also be provided for tapping a sample of combustion product.

The measuring cell 60 is preferably a known electrochemical $ZrO_2$ fuel cell, for example of the type disclosed in the above-identified US Patent No. 3 597 345.

A signal generated by the measuring cell 60 is processed by a circuit 36. The circuit 36 analyses the signal from the measuring cell 60 and is operable to determine the amount of oxygen remaining in the combustion mixture. Since the amount of oxygen initially supplied by the constant flow pump 18 is known, as is also the amount of test sample or fuel supplied by the constant flow pump 22, the amount of oxygen actually consumed in the catalytic combustion chamber 10 can be calculated, which oxygen amount is proportional to the calorific value of the test gas.

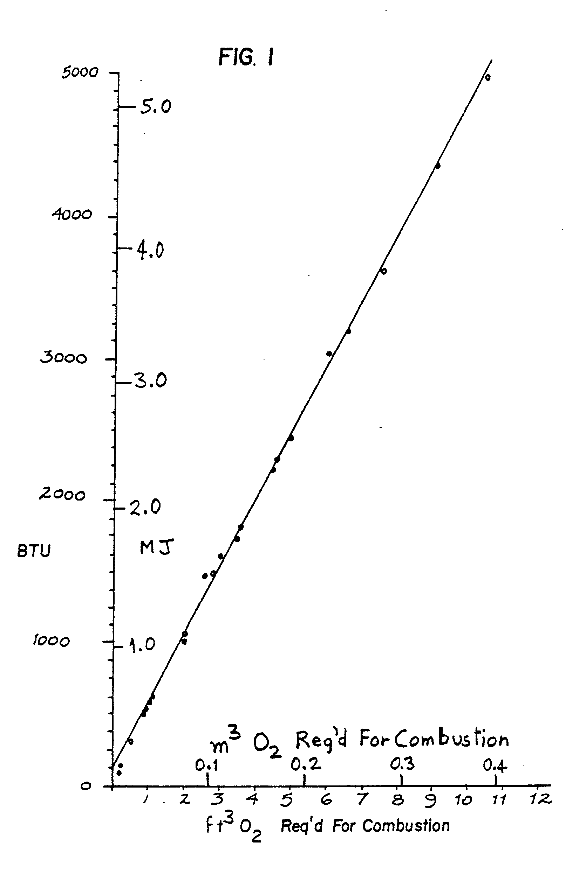

Figure 1 demonstrates the linear relationship between calorific value in MJ (BTU) and amount of oxygen utilized in the combustion of various combustible gases or fuels. As shown in a Table given below, the calorific value of various combustible gases is directly proportional to the amount of oxygen needed to completely burn the same amount of the test gas. These values and other relevant information can be obtained from Handbook of Chemistry, N. A. Lange, Handbook Publishers, Inc., Sandusky, Ohio, 1952, pages 802—803.

It is noted that more than enough air must be provided by the pump 18 to completely burn all oxidisable components of the sample gas supplied by the pump 22. This ensures the maintenance of residual oxygen in the combustion products.

Since the amount of required oxygen is not necessarily dependent on the hydrogen-carbon ratio of the fuel or test gas, the above-described arrangement is usable for a wide variety of fuels, for example as set forth in the following Table.

3

# 0 098 716

TABLE

COMBUSTION CONSTANTS OF GASES

| Name | MJ/m³ (BTU/ft³), gross 15.6°C (60°F) 101.6 kPa (30 in Hg), satd. $H_2O$ | Volume (e.g. m³ or ft³) $O_2$ Req'd. per unit volume (e.q. m³ or ft³) of gas |
|---|---|---|
| Acetylene | 54.21 (1456) | 2.5 |
| Benzene | 136.20 (3658) | 7.5 |
| Butane | 119.30 (3204) | 6.5 |
| Butylene | 112.93 (3033) | 6.0 |
| Carbon Monoxide | 11.81 (317.1) | 0.5 |
| Ethane | 64.45 (1731) | 3.5 |
| Ethylene | 60.06 (1613) | 3.0 |
| Hydrogen | 11.87 (318.8) | 0.5 |
| Methane | 37.05 (995) | 2.0 |
| Propane | 91.78 (2465) | 5.0 |
| Propylene | 86.12 (2313) | 4.5 |
| Toluene | 162.49 (4364) | 9.0 |
| Xylene | 188.55 (5064) | 10.5 |
| Blast Furnace Gas | 3.46 (93) | 0.14 |
| Blue Water Gas | 11.54 (310) | 0.456 |
| Carburetted Water Gas | 21.52 (578) | 0.970 |
| Coal Gas | 23.61 (634) | 1.100 |
| Coke Oven Gas(1) | 19.96 (536) | 0.930 |
| Coke Oven Gas(2) | 22.34 (600) | 1.056 |
| Natural Gas (Follansbee, W.Va.) | 82.66 (2220) | 4.300 |
| Natural Gas Residual (Follansbee, W.Va.) | 69.55 (1868) | 3.594 |
| Natural Gas (McKean Co., Pa.) | 55.18 (1482) | 2.850 |
| Natural Gas (Sandusky, Ohio) | 38.98 (1047) | 2.008 |
| Oil gas | 19.21 (516) | 0.850 |
| Producer Gas | 5.06 (136) | 0.216 |

Catalytic burning is preferred over flame burning due to the possibility of completely burning all combustible products in the test sample. To overcome a possible problem in catalyst poisoning, a relatively large catalytic bed is preferably utilised in the chamber 10 to prolong catalyst life.

According to the above-described technique, calorific values can be obtained rapidly, without the delay which is usual in known calorimeters.

**Claims**

1. A calorimeter for continuously monitoring the calorific value of a test gas, the calorimeter (50) comprising:

first flow means (22) for supplying a known volume of test gas;

second flow means (18) for supplying a known volume of oxygen-containing gas having a known oxygen amount;

a burning chamber (10) connected to the first and second flow means (22, 18) for receiving the test and oxygen-containing gases and completely burning the test gas with an amount of oxygen of the oxygen-containing gas to produce a combustion product;

heating means (14) for heating the burning chamber (10) to produce burning;

an electrochemical oxygen measuring cell (60) connected to the chamber (10) for receiving the combustion product and measuring any remaining oxygen in the product; and

circuit means (36) connected to the cell (60);

characterised in that:

the first and second flow means (22, 18) are constant flow means for respectively supplying constant known volumes of test gas and oxygen-containing gas;

the burning chamber is a catalytic burning chamber (10);

the constant known volumes of the test gas and the oxygen-containing gas are set so that the combustion product has residual oxygen therein; and

the circuit means is operative to generate a value proportional to the difference between the known volume of oxygen and the amount of remaining oxygen, which value is proportional to the amount of oxygen consumed in the combustion chamber (10), the amount of oxygen consumed being proportional to the calorific value of the test gas.

2. A calorimeter according to claim 1, wherein the first and second constant flow means comprise first and second constant flow pumps (22, 18).

3. A calorimeter according to claim 2, wherein the first and second constant flow pumps (22, 18) comprise reciprocating cylinder pumps.

4. A calorimeter according to claim 1, claim 2 or claim 3, wherein the electrochemical oxygen measuring cell (60) comprises a $ZrO_2$ fuel cell.

5. A method of continuously monitoring the calorific value of a test gas, the method comprising:

supplying the test gas at a known flow rate to a combustion chamber (10);

supplying an oxygen-containing gas having a known oxygen amount to the combustion chamber (10) at a known flow rate;

heating the combustion chamber (10) to burn the test gas to consume an amount of oxygen and produce a combustion product;

measuring the amount of any remaining oxygen in the combustion product; and

determining the calorific value of the test gas;

characterised in that:

the test gas and the oxygen-containing gas are supplied at constant known flow rates;

the combustion chamber is a catalytic combustion chamber (10);

the constant known flow rates of the test gas and the oxygen-containing gas are set so that the combustion product has residual oxygen therein; and

the calorific value of the test gas is determined from the measured amount of oxygen consumed in the catalytic burning chamber (10), which amount of oxygen consumed is derived from the difference between the known volumetric flow of oxygen into the chamber and the amount of residual oxygen in the combustion product.

**Patentansprüche**

1. Kalorimeter zur fortlaufenden Überwachung des Heizwertes eines Prüfgases, wobei das Kalorimeter (50) aufweist:

erste Fließmittel (22) für die Zufuhr eines bekannten Volumens an Prüfgas;

zweite Fließmittel (18) für die Zufuhr eines bekannten Volumens an Sauerstoff enthaltendem Gas mit einer bekannten Sauerstoffmenge;

eine Brennkammer (10), die mit dem ersten und zweiten Fließmittel (22, 18) für die Aufnahme des Prüfgases und des Sauerstoff enthaltenden Gases und für die vollständige Verbrennung des Prüfgases mit einer Sauerstoffmenge des Sauerstoff enthaltenden Gases verbunden ist, um ein Verbrennungsprodukt zu erzeugen;

Heizmittel (14) zur Erwärmung der Brennkammer (10) zur Erzeugung der Verbrennung;

eine elektrochemische Sauerstoffmeßzelle (60), die mit der Kammer (10) verbunden ist zur Aufnahme des Verbrennungsproduktes und Messung von etwaig verbleibendem Sauerstoff in dem Produkt; und

Schaltmittel (36), die mit der Zelle (60) verbunden sind;

dadurch gekennzeichnet, daß

das erste und zweite Fließmittel (22, 18) Konstantströmungsmittel sind zur jeweiligen Zuführung konstanter, bekannter Volumina an Prüfgas und Sauerstoff enthaltendem Gas;

die Brennkammer eine katalytische Brennkammer (10) ist;

die konstanten, bekannten Volumina des. Prüfgases und des Sauerstoff enthaltenden Gases so eingestellt werden, daß in dem Verbrennungsprodukt ein Rest Sauerstoff ist; und

das Schaltmittel im Betrieb einen Wert proportional zur Differenz zwischen dem bekannten Volumen an Sauerstoff und der Menge an verbleibendem Sauerstoff erzeugt, wobei dieser Wert proportional ist zur Menge an in der Verbrennungskammer (10) verbrauchtem Sauerstoff und die Menge an verbrauchtem Sauerstoff proportional zum Heizwert des Prüfgases ist.

2. Kalorimeter nach Anspruch 1, wobei das erste und zweite Konstantströmungsmittel erste und zweite Konstantströmungspumpen (22, 18) aufweisen.

3. Kalorimeter nach Anspruch 2, wobei die erste und zweite Konstantsrömungspumpe (22, 18) sich hin- und herbewegende Zylinderpumpen aufweisen.

4. Kalorimeter nach Anspruch 1, Anspruch 2 oder Anspruch 3, wobei die elektrochemische Sauerstoffmeßzelle (60) eine $ZrO_2$ Brennstoffzelle ist.

5. Verfahren zur kontinuierlichen Überwachung des Heizwertes eines Prüfgases, wobei das Verfahren aufweist:

das Zuführen von Prüfgas mit einer bekannten Fließgeschwindigkeit zu einer Verbrennungskammer (10);

Zuführen eines Sauerstoff enthaltenden Gases mit einer bekannten Sauerstoffmenge zu der Brennkammer (10) hin mit einer bekannten Fließgeschwindigkeit;

Erwärmung der Brennkammer (10) zur Verbrennung des Prüfgases für den Verbrauch einer Menge Sauerstoff und Erzeugung eines Verbrennungsproduktes;

Messen der Menge von etwa verbleibendem Sauerstoff in dem Verbrennungsprodukt; und Bestimmung des Heizwertes des Prüfgases;

dadurch gekennzeichnet, daß:

das Prüfgas und das Sauerstoff enthaltende Gas mit konstanten, bekannten Fließgeschwindigkeiten zugeführt werden;

die Brennkammer eine katalytische Brennkammer (10) ist;

die konstanten, bekannten Fließgeschwindigkeiten des Prüfgases und des Sauerstoff enthaltenden Gases so eingestellt werden, daß in dem Verbrennungsprodukt ein Rest Sauerstoff ist; und

der Heizwert des Prüfgases bestimmt wird aus der gemessenen Menge an Sauerstoff, der in der katalytischen Brennkammer (10) verbraucht ist, wobei die Menge an verbrauchtem Sauerstoff abgeleitet wird von der Differenz zwischen der bekannten volumetrischen Strömung an Sauerstoff in die Kammer hinein und der Menge an Restsauerstoff im Verbrennungsprodukt.

**Revendications**

1. Calorimètre pour la surveillance en continu du pouvoir calorifique d'un gaz d'essai, le calorimètre (50) comprenant:

de premiers moyens d'écoulement (22) pour l'amenée d'un volume connu de gaz d'essai;

de seconds moyens d'écoulement (18) pour l'amenée d'un volume connu de gaz contenant de l'oxygène ayant une teneur en oxygène connue;

une chambre de combustion (10) reliée aux premiers et seconds moyens d'écoulement (22, 18) pour recevoir le gaz d'essai et celui contenant de l'oxygène et brûler complètement le gaz d'essai avec une certaien quantité d'oxygène du gaz contenant de l'oxygène afin de donner un produit de combustion;

des moyens de chauffage (14) pour chauffer la chambre de combustion afin de provoquer la combustion;

une cellule électrochimique de mesure d'oxygène (60) reliée à la chambre (10) pour recevoir le produit de combustion et mesurer le reste éventuel d'oxygène qu'il contient; et

des moyens formant circuit (36) reliés à la cellule (60); caractérisé en ce que:

les premiers et seconds moyens d'écoulement (22, 18) sont des moyens à débit constant afin d'amener respectivement des volumes connus constants de gaz d'essai et de gaz contenant de l'oxygène;

la chambre de combustion est une chambre de combustion catalytique (10);

les volumes connus constants du gaz d'essai et du gaz contenant de l'oxygène sont réglés en sorte que le produit de combustion contienne. de l'oxygène résiduel; et

le moyen formant circuit assure la génération d'une valeur proportionnelle à la différence entre le volume connu d'oxygène et a quantité d'oxygène restant, laquelle valeur est proportionnelle à la quantité d'oxygène consommé dans la chambre de combustion (10), la quantité d'oxygène consommée étant proportionnelle au pouvoir calorifique du gaz d'essai.

2. Calorimètre selon la revendication 1, dans lequel les premiers et seconds moyens à débit constant sont une première et une seconde pompes à débit constant (22, 18).

3. Calorimètre selon la revendication 2, caractérisé en ce que les première et seconde pompes à débit constant (22, 18) sont des pompes à cylindre à mouvement alternatif.

4. Calorimètre selon la revendication 1, la revendication 2 ou la revendication 3, dans lequel la cellule électrochimique de mesure d'oxygène (60) est une cellule électrochimique au $ZrO_2$.

5. Procédé de surveillance en continu du pouvoir calorifique d'une gaz d'essai, le procédé comprenant:

l'amenée du gaz d'essai à un débit connu à une chambre de combustion (10);

l'amenée à la chambre de combustion (10) à un débit connu d'un gaz contenant de l'oxygène comportant une quantité connue d'oxygène;

le chauffage de la chambre de combustion (10) opéré pour brûler le gaz d'essai afin de consommer une certaine quantité d'oxygène et d'obtenir un produit de combustion;

la mesure de la quantité d'oxygène subsistant éventuellement dans le produit de combustion; et

la détermination du pouvoir calorifique du gaz d'essai;

caractérisé en ce que:

le gaz d'essai et le gaz contenant de l'oxygène sont amenés à des débits connus constants;

la chambre de combustion est une chambre de combustion catalytique (10);

les volumes connus constants du gaz d'essai et du gaz contenant de l'oxygène sont réglés en sorte que le produit de combustion contienne de l'oxygène résiduel; et

le pouvoir calorifique du gaz d'essai est déterminé d'après la quantité mesurée d'oxygène consommée dans la chambre de combustion catalytique (10), laquelle quantité d'oxygène est déduite de la différence entre le débit volumétrique connu de pénétration d'oxygène dans la chambre et la quantité d'oxygène résiduel présente dans le produit de combustion.

FIG. 1

FIG. 2